# EUROPEAN PATENT APPLICATION

(11) **EP 0 594 282 A2**
(43) Date of publication of application: **27.04.1994**
(21) Application number: 93305201.1
(22) Date of filing: 02.07.1993
(51) Int. Cl.: C12M 3/00

(54) **Electrode for electrical cell fusion and electrical introduction of nucleic acids**

(30) Priority: 29.07.1992 JP 202707/92
(71) Applicant: DIRECTOR GENERAL OF FRUIT TREE RESEARCH STATION OF JAPAN, MINISTRY OF AGRICULTURE, FORESTRY AND FISHERIES, Tsukuba, Ibaraki (JP)
(72) Inventor: Omura, Mitsuo, Shizuoka, Shizuoka (JP); Hidaka, Tetsushi, Shimizu, Shizuoka (JP)
(74) Representative: Senior, Alan Murray

(57) **Abstract**

This aim of this invention is to make possible efficient, large-scale cell fusion and introduction of nucleic acids by the electrical method.

An electrode of which a contact part has an overall cross-section matching the shape of the base of a cylindrical container for cell culture, and wherein electrical cell fusion and electrical introduction of nucleic acids are performed by applying an A.C. voltage to a plurality of conductors so as to generate an electrical field, and applying a D.C. pulse in synchronism, is characterized in that these conductors are disposed such that the cross-section of the contact part consists of a series of concentric rings formed by the conductors, in that the conductors have flat surfaces, and in that the conductors or their surfaces consist of a stable material which does not easily ionize.

## Description

This invention relates to an electrode having a novel construction which permits cell fusion and introduction of nucleic acids to be carried out efficiently by means of an electrical method.

Recently, in order to improve varieties of plant and animal species, attempts have been made to perform cell fusion not only between cells of the same type but also between cells of different type.

These attempts can be divided mainly into chemical methods using chemical substances such as polyethylene glycol, and an electrical method using electrical pulses.

Chemical methods are traditional, but are associated with the following disadvantages:
1) the chemical substances used when fusion is performed have cell toxicity,
2) it is difficult to set optimum fusion conditions,
3) skill is required to perform the fusion technique,
4) the fusion efficiency is generally rather low (3 - 5%).

The electrical method, on the other hand, does not present any risk that cells will be damaged by contact with specific chemical substances, does not call for any technique requiring special operating skill, is simple to perform, and permits cell fusion to be performed with a high degree of efficiency.

The electrical method was conceived by Senda et al, who discovered in 1979 that cells fused when subjected to a D.C. pulse, and it was established as an independent technique by Melchers in Germany (West Germany at that time).

Electrical introduction of nucleic acids was first reported by Newmann in 1982. This was achieved by mixing protoplast with a nucleic acid, and applying a D.C. pulse.

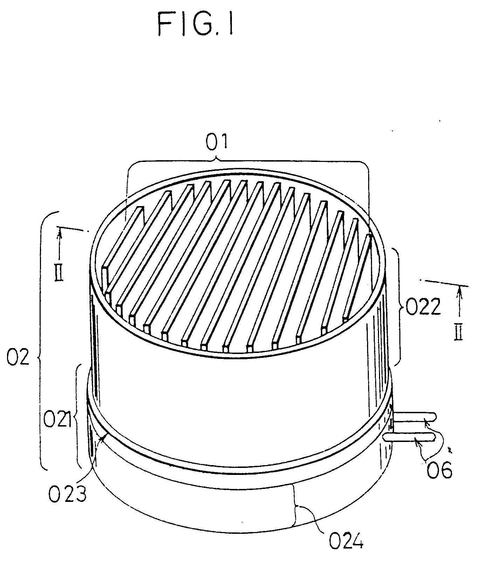

Figs. 1 - 4 show the form of electrodes conventionally used for a typical cell fusion or introduction of nucleic acids. These will now be described in further detail.

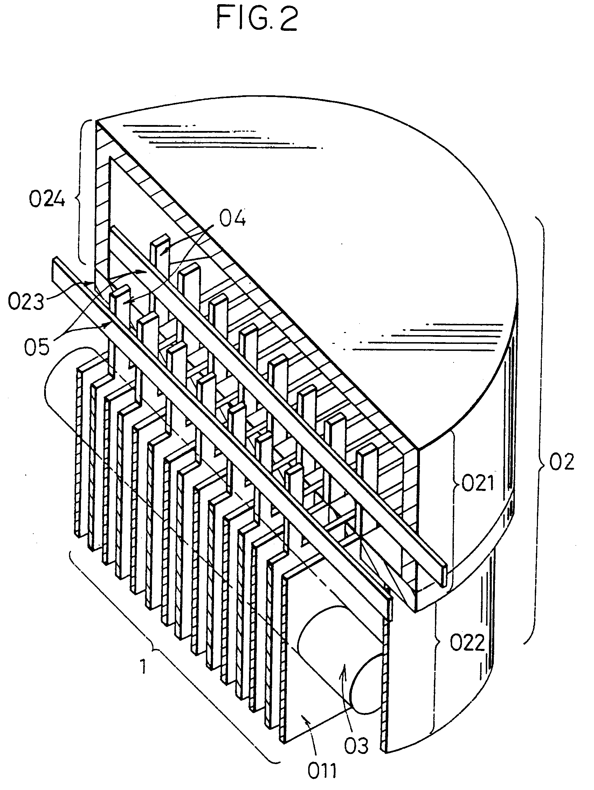

(1) Fig. 1 is a general perspective view of one type of electrode used to fuse cells in a Petri dish and introduce nucleic acids into these cells. Fig. 2 is an enlarged view of the essential parts of Fig. 1.

In Fig. 1, the electrode consists of a series of conducting plates 01 arranged parallel to one another which are supported by an envelope 02 having a circular cross-section open at one end.

Fig. 2 shows this electrode in further detail.

In Fig. 2, the conducting plates 01 are square plates formed of a conducting material. Holes which fit the cross-sectional shape of a column-shaped member 03 described hereinafter are provided at the intersection of the diagonals in the plane of these electrode plates. Further, rectangular projections 04 are provided near the middle of one side of each square plate at equal distances from this middle point (hereinafter, the direction corresponding to one side of a component member 011 of the plates 01 provided with a rectangular projection 04 is referred to as the "width direction", and the direction perpendicular to this width direction is referred to as the "length direction"). The thickness of the conducting plates in the length direction is the same for all the plates. On the other hand, the length of each plate in the width direction is different, and depends on the length of arc cut off by the plate on the circumference of a contact part 022 of a cylindrical housing described hereinafter.

Next, a circular plate 023 forming the lower surface of a suspension part of the cylindrical housing, is inserted from the direction of the rectangular projections 04 so as to fix the assembly near the base of the projections 04. This plate 023 has parallel, equidistant grooves, the length of each groove corresponding to the length of arc cut off by the groove on the circumference of the contact part 022 of the cylindrical housing, the width of each groove being such as to accommodate the thickness of each electrode plate, and the number of these grooves depending on the number of electrode plates. The projections 04 are arranged in a staggered fashion such that projections on alternate plates are aligned in front of each other.

Rod-shaped conductors 05 are provided at an interval corresponding to the distance in the width direction between a plane formed by one row of the projections 04 perpendicular to the width direction of each member 011 comprising the electrode plates 01, and a similar plane formed by the other row of the projections 04.

The rod-shaped conductors 05 pass through a lateral surface of the housing such that one end of each projects from the surface, and a cylindrical body forming the suspen sion part having one open end is covered by the circular plate 023 which has the same diameter so as to form the cylindrical housing. The conductors 05 are fixed in contact with the lateral surfaces of the rectangular projections 04 by a means which permits passage of electrical current.

The column-shaped member 03, which consists of a non-conducting material, has a cross-section which matches the shape of the holes in the conducting plates, and it is made to pass through the plates. Its two ends are firmly held by the inner surfaces of the contact part 022 of the cylindrical housing, and are bonded to them when the contact part 022 is bonded to one end of the suspension part 021.

When it is desired to perform cell fusion using this electrode assembled as described hereintofore, the contact part of the cylindrical housing is immersed in a cell suspension in a Petri dish, and a high frequency A.C. voltage is applied via connectors 6. Electrical migration occurs between the electrode plates, and a "pearl chain" of cells is set up between the plates. A D.C. voltage is then applied pulsewise, and parts of cells in contact with one another are broken down. The result is that all or part of the cells fuse together. When it is desired to introduce nucleic acids, a D.C. pulse voltage is applied directly to the cells without applying the A.C. voltage. This makes pores in the cell surfaces through which nucleic acids from the suspension enter, and the desired introduc tion of nucleic acids therefore occurs.

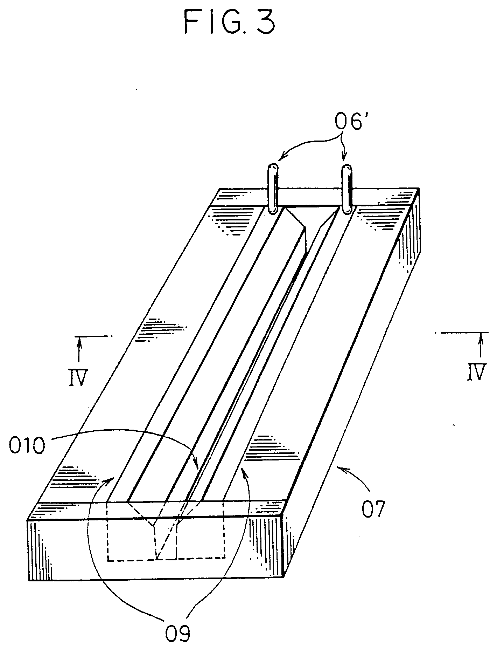

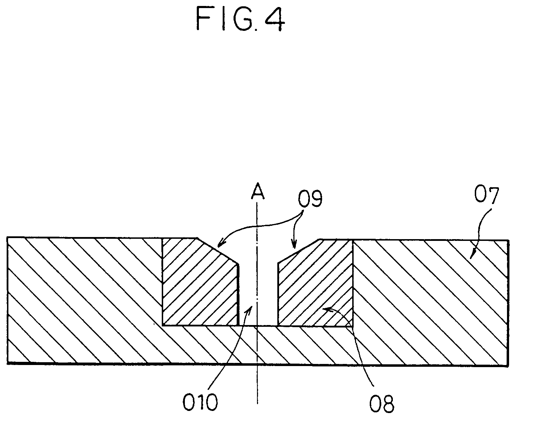

(2) Fig. 3 is a front view of a cell fusion/nucleic acid introduction device using a parallel electrode method wherein the electrodes have been integrated with a cell fusion Chamber. Fig. 4 is a cross-sectional view of the same device.

In Figs. 3 and 4, a rectangular groove 08 is formed in a longitudinal direction in the center of the upper surface of a block having a rectangular cross-section 07. Electrodes 09 having a trapezoidal cross-section which fits the lateral walls and part of the base of this groove 08, an inclined upper side, and a perpendicular side parallel to the aforesaid lateral walls, are symmetrically disposed in a longitudinal direction opposite each other on either side of a center line A.

A pair of pins used as connectors consisting of an electrically conducting material are connected to the upper parts of these electrodes, one pin to one electrode, by electrically conducting means.

A space 010 of rectangular cross-section is thereby formed by the electrodes 09 and the groove 08, this space widening toward its upper side and being formed in the longitudinal direction of the groove 08.

When it is desired to perform cell fusion using this electrode assembly, a cell suspension is introduced into the space 010, and a high frequency A.C. voltage is applied to the electrodes via the connectors 06 which are directly connected to them. Electrical migration occurs between the electrodes, and a "pearl chain" is set up between the cells in the space 010. A D.C. voltage is then applied pulsewise, and parts of cells in contact with one another are broken down. The result is that all or part of the cells fuse together. When it is desired to introduce nucleic acids, a D.C. pulse voltage is applied directly to the cells without applying the A.C. voltage. This makes pores in the cell surfaces through which nucleic acids from the suspension enter, and the desired introduction of nucleic acids therefore occurs.

The aforesaid conventional electrodes used for cell fusion and introduction of nucleic acids were however associated with the following problems.

The electrode shown in Figs. 1 and 2 has an advantage in that, as the electrodes come into direct contact with the cell suspension in the Petri dish, cells in a plurality of Petri dishes can be fused in succession one after another. However, due to the relation between the circular cross-section of the contact part of the aforesaid cylindrical housing and the electrode plates disposed parallel to each other, the electrode plate closest to the circumference cuts off a crescent-shaped segment in the cross-sectional plane of the contact part. In this crescent-shaped segment, cell fusion cannot take place uniformly, and it consequently cannot be performed efficiently.

Moreover, in the cell fusion device using parallel electrodes shown in Figs. 3 and 4, although is no problem of the aforesaid type (1), the cell suspension in the space 010 has to be replaced after each cell fusion operation, and this is a disadvantage from the viewpoint of operating efficiency when it is required to treat large volumes of suspension.

Furthermore, in (1) and (2) above, the electrode plates consist of a conducting material which has received no surface treatment. The electrode plates have small surface imperfections so that when an A.C. voltage or a D.C. pulse voltage is applied, the electrode field set up between the electrodes is uneven, and this has an adverse effect on cell fusion and introduction of nucleic acids. More particularly, in cell fusion, the "pearl chain" cannot form evenly due to non-uniformity of the A.C. electric field, and the fusion efficiency of the cell fusion process falls due to non-uniformity of the D.C. electric field. Further, in introduction of nucleic acids, the efficiency of introducing nucleic acids falls due to non-uniformity of the D.C. electric field.

The Inventors, after intensive studies, successfully resolved the aforesaid problems by providing the invention described hereinbelow.
(1) An electrode for performing electrical cell fusion and electrical introduction of nucleic acids wherein an A.C. voltage and a D.C. pulse voltage are applied either simultaneously or separately to a plurality of electrical conductors capable of interacting with each other so as to produce an electrical field, this electrode being characterized in that the electrical conductors have flat surfaces, and in that the electrical conductors or their surfaces consist of a stable material which does not easily ionize.
(2) An electrode as described in (1) characterized in that the stable material which does not easily ionize is gold or platinum, and in that the surfaces of the electrical conductors are mirror polished.
(3) An electrode for performing electrical cell fusion and electrical introduction of nucleic acids wherein an A.C. voltage and a D.C. pulse voltage are applied either simultaneously or separately to a plurality of electrical conductors capable of interacting with each other so as to produce an electrical field, the cross-section of a contact part having a shape which matches the base of a cylindrical container used for cell culture, and the electrical conductors being so arranged that the cross-section of the contact part consists of concentric rings of the conductors.
(4) An electrode as described in (3) characterized in that the electrical conductors forming concentric rings have flat surfaces, and in that the electrical conductors or their surfaces consist of a stable material which does not easily ionize.
(5) An electrode as described in (4) characterized in that the stable material which does not easily ionize is gold or platinum, and in that the surfaces of the electrical conductors are mirror polished.

In the context of this invention, the expression "an electrode for performing electrical cell fusion and electrical introduction of nucleic acids wherein an A.C. voltage and a D.C. pulse voltage are applied either simultaneously or separately to a plurality of electrical conductors capable of interacting with each other so as to produce an electrical field", refers to an electrode as for example described in "Background of the Invention" which is shown in Figs. 1, 2, 3 or 4, there being no particular limitation on the form of this electrode provided that it satisfies the aforesaid conditions.

In the present context, the expression "a plurality of electrical conductors" refers to the part corresponding to 01 in Fig. 1 or 09 in Fig. 3. Further, the A.C. voltage applied when it is desired to perform cell fusion must be of a frequency and magnitude suitable for forming a "pearl chain" wherein the cells to be fused are arranged in a straight line. The fusion conditions are selected depending on the type of cells to be fused, i.e. plant or animal, and on their species and varieties. In the case of plants, for example, the frequency is generally about 1 MHz and the magnitude is of the order of 50 - 200V/cm, although these conditions are not limiting. The D.C. pulse current required to be applied for both cell fusion and introduction of nucleic acids must have a voltage, pulse length, application frequency and pulse interval capable of making pores in the cell surfaces without causing any serious damage to them. As in the case of the aforesaid A.C. voltage, the conditions for applying a D.C. voltage in cell fusion and introduction of nucleic acids are suitably chosen depending on the type of cells to be fused or in which nucleic acids are to be introduced. For fusing plant cells, for example, a rectangular wave having a voltage of 800 - 2000V/cm and a pulse width of about 50 - 300 µ seconds is generally used. For introducing nucleic acids, a D.C. pulse of greater length than that used for cell fusion may be used in the form of a rectangular wave or an attenuated wave. For introducing nucleic acids in plant cells, for example, a D.C. pulse having a width of about 1 M seconds may be used in the form of a rectangular wave or an attenuated wave. As in the case of A.C. voltages, these conditions are not necessarily limiting. The device used for applying this A.C. voltage and D.C. pulse voltage either simultaneously or separately may consist of a combination of an oscillator which permits control of the magnitude and frequency of the aforesaid A.C. voltage, and an oscillator which permits control of the magnitude, pulse count and pulse width of the aforesaid D.C. pulse voltage. Alternatively, it may consist of an oscillator having both of these functions. For cell fusion, both voltages are applied simultaneously, and for introducing nucleic acids, only a D.C. pulse current is applied.

The expression "the electrical conductors have flat surfaces" means that the surfaces of the conductors are as flat as possible. More specifically, the surfaces may be of a metal such as stainless steel, iron or aluminum which is polished, coated with a noble metal such as gold or platinum, and then smoothed by mirror polishing. There is no particular limitation on the coating method used, typical methods being ion plating, vapor deposition, CVD and MVD. If a method is adopted wherein the surface is coated with a noble metal, the substrate does not necessarily have to be a conducting metal, it being possible to use also non-metals such as glass, quartz or plastic as the substrate. Further, examples of a "stable material which does not easily ionize" are the aforesaid noble metals such as gold and platinum, and their alloys.

Next, the expression "cylindrical container used for cell culture" according to claim 3 of the invention, refers to a container for cell culture having a circular cross-section, a typical example being a Petri dish. The expression "contact part" refers to a part with which a solution containing cells which are to be fused, or in which nucleic acids are to be introduced, comes into direct contact. The expression "concentric rings" includes not only concentric rings spaced at equal intervals apart, but also concentric rings spaced at different intervals apart.

There is no particular limitation on the type of cells which can be fused or in which nucleic acids can be introduced using the electrode of this invention, the cells which can be fused covering a wide range such as plant cells, animal cells, bacteria, fungus and yeast. Depending on the nature of the cells, however, it may be necessary to subject the cells to a pre-treatment prior to electrical fusion using the electrode of this invention. In the case of fusion of plant cells, for example, a protoplast treatment is first performed to remove the cell wall.

According to this invention, the electrical conductor has a flat surface, and either the conductor or its surface consists of a stable material which does not easily ionize such as gold or platinum. Distortions of the electrical field due to the presence of minute surface imperfections are thereby corrected, mutual interactions between the cell surface and the conductor surface are lessened, and cell adsorption on the conductor surface is reduced.

Further, according to this invention, the electrical conductors are disposed in such a manner that the cross-section of the aforesaid contact part consists of concentric rings of conductors, and redundant spaces in the contact part which the electrical field does not affect are thereby reduced.

Due to the aforesaid construction of the invention and its attendant advantages, the electrical method can be used to perform cell fusion and introduction of nucleic acids efficiently and on a large scale.

The present invention will be further described hereinafter with reference to the following description of exemplary embodiments and the accompanying drawings, in which:
Fig. 1 is an overall perspective view of one form of an electrode used to perform cell fusion or introduce nucleic acids into cells in a Petri dish.
Fig. 2 is an enlarged view of the essential parts of the electrode shown in Fig. 1.
Fig. 3 is a front view of a cell fusion device using a parallel electrode method wherein the electrodes are integrated with the cell fusion Chamber in a one-piece construction.
Fig. 4 is a sectional view of the device shown in Fig. 3.

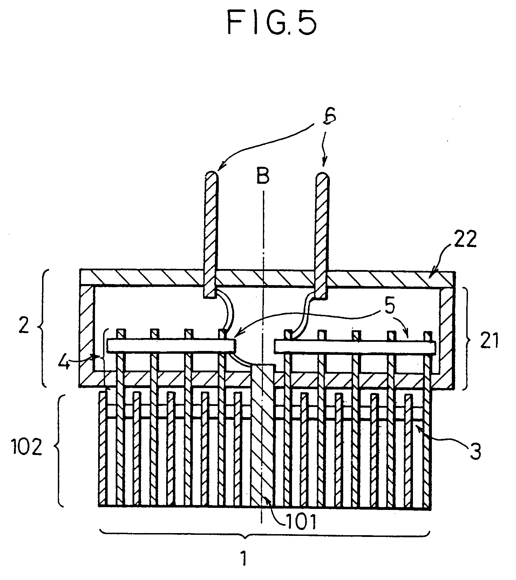

Fig. 5 is a sectional view of one form of the electrodes according to this invention.

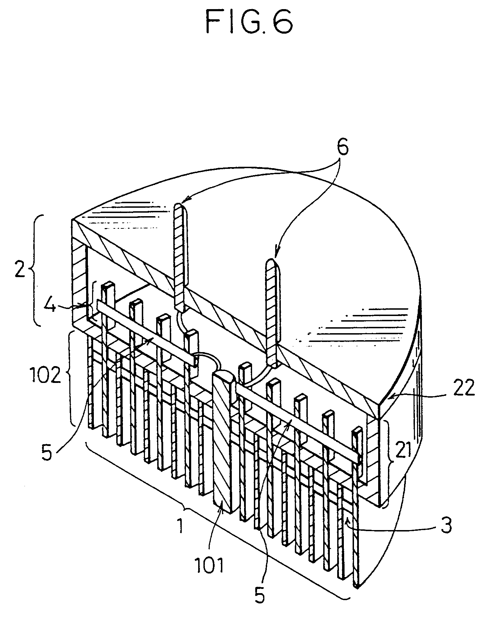

Fig. 6 is an enlarged view of the essential parts of the electrode shown in Fig. 5.

Figs. 5 and 6 are drawings showing one form of the electrode according to this invention. Fig. 5 is a cross-sectional view of this electrode, and Fig. 6 is an enlarged view of its essential parts.

First, in Fig. 5, conducting cylinders 1 consisting of an electrically conducting material and having two open ends, are each provided with two holes at symmetrical positions on either side of a central line B of the cylinders, these holes matching the shape of the cross-section of a column 3 described hereinafter. Rectangular projections 4 are also provided on one end of all the conducting cylinders. These cylinders 1 are so designed that their heights are all the same, but their diameters are shorter than the diameter of the cylindrical housing 2 and the difference between the diameters of adjacent cylinders is constant. The surface of each cylinder 1 is gold-plated to give it a mirror surface.

Next, the aforesaid conducting cylinders 1 are arranged in concentric rings. The projection on each cylinder 1 is disposed on the opposite side of the center line B with respect to the projection on the next cylinder further out with the result that the projections are arranged in a straight line. A cylinder 21 of which one end is closed by a circular plate forming the base of the cylindrical housing 2, and provided with holes which fit the cross-sections of the projections at positions corresponding to the row of projections on the cylinders 1, is inserted into the cylinders 1 from the direction of the projections with the open end of the cylinder 21 oriented upward so as to fix the bases of the projections. Before performing this operation, however, a column-shaped support 101 formed from an electrical conductor is first passed through and fixed in the center point of the circular plate perpendicular to its plane.

Next, rod-shaped members 5 consisting of separate electrical conductors are brought into contact with the lateral surfaces of the projections on either side of the center line B such that they are parallel to the base of the cylindrical housing 2, and fixed by means which permit passage of electrical current. The rod-shaped member 5 different from the rod-shaped member in contact with the projection 4 closest to the center of the circular plate, is connected to the support 101 by a means which permits passage of electrical current.

Next, a lid 22 through which pass two rod-shaped connectors 6 consisting of electrical conductors, and having a shape matching the shape of the base of the aforesaid cylinder 21, is engaged with the cylinder 21. The arrangement is such that one of the aforesaid rod-shaped connectors 6 is connected by a means which permits passage of electrical current to one of the connectors 5 which connects conducting cylinders together, and the other connector 6 is connected by a similar means to the other connector 5 which connects conducting cylinders together.

Finally, column-shaped members 3 consisting of a non-conductor and having a cross-section which fits the holes provided in the conducting plates, are passed through the conducting plates 1, and are firmly held in place by the aforesaid column-shaped support 101.

In the electrode of this invention for performing cell fusion or introducing nucleic acids assembled as described hereintofore, a high frequency A.C. voltage is applied via the connectors 6 to a contact part 102 designed to match the shape of a Petri dish with which it comes into contact.

A "pearl chain" is set up in the cells between the conducting cylinders 1 which are arranged concentrically.

Next, this voltage is applied under the same conditions via the connectors 6 in the form of a pulse so as to break down the parts of cells in contact with one another. As a result, some or all of the cells fuse together. In order to introduce nucleic acids, a D.C. voltage is applied directly to the cells without applying the aforementioned A.C. voltage so as to make pores in the cell surfaces through which nucleic acids in suspension can enter. The desired introduction of nucleic acids therefore takes place. The above description of an embodiment of the invention should not be construed as meaning that the invention is limited to it in any way, and various design modifications are possible within the scope of the claims appended thereto.

## Claims

1. An electrode for performing electrical cell fusion and electrical introduction of nucleic acids wherein an A.C. voltage and a D.C. pulse voltage are applied either simultaneously or separately to a plurality of electrical conductors capable of interacting with each other so as to produce an electrical field, said electrode being characterized in that said electrical conductors have flat surfaces, and in that said electrical conductors or their surfaces consist of a stable material which does not easily ionize.

2. An electrode as defined in claim 1 characterized in that said stable material which does not easily ionize is gold or platinum, and in that the surfaces of said electrical conductors are mirror polished.

3. An electrode for performing electrical cell fusion and electrical introduction of nucleic acids wherein an A.C. voltage and a D.C. pulse voltage are applied either simultaneously or separately to a plurality of electrical conductors capable of interacting with each other so as to produce an electrical field, the cross-section of a contact part having a shape which matches the base of a cylindrical container used for cell culture, and said electrical conductors being so arranged that said contact part consists of concentric rings of said conductors.

4. An electrode as defined in claim 3 characterized in that said electrical conductors forming concentric rings have flat surfaces, and in that said electrical conductors or their surfaces consist of a stable material which does not easily ionize.

5. An electrode as defined in claim 4 characterized in that said stable material which does not easily ionize is gold or platinum, and in that the surfaces of said electrical conductors are mirror polished.

6. An apparatus for performing electrical cell fusion including an electrode according to any one of the preceding claims.

7. An apparatus according to claim 1 further including a cylindrical container within which said electrode is disposed.
